Europäisches Patentamt

European Patent Office

Office européen des brevets

(19)

(11) Publication number: 0 313 274
A1

(12) **EUROPEAN PATENT APPLICATION**

(21) Application number: 88309624.0

(22) Date of filing: 14.10.88

(51) Int. Cl.⁴ **G01N 33/58 , G01N 33/543 ,
G01N 33/74 , G01N 33/533 ,
//G01N33/94**

(30) Priority: 22.10.87 GB 8724764

(43) Date of publication of application:
26.04.89 Bulletin 89/17

(84) Designated Contracting States:
BE CH DE ES FR GB IT LI NL SE

(71) Applicant: **ALCAN INTERNATIONAL LIMITED**
**1188 Sherbrooke Street West**
**Montreal Quebec H3A 3G2(CA)**

(72) Inventor: **Abuknesha, Ramadan Arabi**
**16 Burgess Park Mansion Fortune Green**
**Road**
**West Hampstead London NW6 1DP(GB)**
Inventor: **Bennetto, Hugh Peter**
**64 Woodheyes Road**
**London NW10(GB)**
Inventor: **Mason, Jeremy Richard**
**45 Friar's Place Lane**
**London W3(GB)**
Inventor: **Nugent, Philip Giles**
**April Cottage 16 Albert Road**
**Kingston-upon-Thames Surrey KT1 3DH(GB)**
Inventor: **Stirling, John Laing**
**78 Twyford Avenue**
**London W3(GB)**
Inventor: **Thurston, Christopher Frank**
**26 Ranleigh Road**
**London W5(GB)**

(74) Representative: **Pennant, Pyers et al**
**Stevens, Hewlett & Perkins 5 Quality Court**
**Chancery Lane**
**London, WC2A 1HZ(GB)**

(54) Enzyme assay method.

(57) An immunoassay method involves the use of one or preferably two enzyme amplification steps to increase assay speed or sensitivity. In a first step, a first enzyme is generated in an amount related to the concentration of an analyte in a sample. In a second step, the first enzyme acts on a first substrate to reveal antigenic determinants of a first product. In a third step, the first product is reacted with an antibody. The extent of this immune reaction is determined, preferably by means of an enzyme signal system. Two assays for digoxin and one for 17α-hydroxyprogesterone all using beta-galactosidase as the first enzyme are described.

## ENZYME ASSAY METHOD

Competition assays, such as immunoassays and immunometric assays, are widely used in clinical chemistry. They afford a level of specificity and sensitivity which is difficult, or impossible, to match by other analytical procedures. There is a need, however, to increase the sensitivity of such assays still further, for two reasons. Firstly, some analytes of interest are present at extremely low concentrations. Secondly, more sensitive assays can commonly be employed to detect higher concentrations of analytes more quickly. Attempts have been made to maximise assay sensitivity by employing radiolabelled, fluorescent or chemiluminescent reagents, but ideal combinations of sensitivity, reproducibility and convenience have not been achieved for all applications.

An alternative strategy to improve assay sensitivity is to employ an amplification step, such that a product which may be generated in not more than stoichiometric amounts to the analyte, generates the production of a significantly higher concentration of a second product which is then observed. The use of enzymes for this purpose has been widely described in the literature. By repeatedly acting as catalysts, enzymes can achieve amplification by factors of hundreds or thousands.

In principle, it is possible to achieve further degrees of amplification by the use of two enzyme systems in sequence. In practice, however, this is not easy to achieve. A.Johannsson et al. (Journal of Immunological Methods, 87 (1986) 7 to 11) describes an assay for TSH. A first catalytic step involves the dephosphorylation of NADP to produce NAD, which catalytically activates a specific redox cycle to generate an intensely coloured compound which is observed. The method has the disadvantage of using esoteric unstable enzymes, and of involving NADP/NAD, so that it cannot be used with samples containing these materials.

Yolken R.H. (Rev. Infec. Dis., 4, (1982) 35-68) describes an immunoassay with complement mediated amplification. The method is dependent on non-specific complement cascade activation steps, and the components are difficult to isolate.

EPA 75379 (Syva) and EPA 152254 (Du Pont) both describe immunoassays which involve binding two enzymes to a solid phase, one in proportion to the analyte concentration, the product of one enzyme being the substrate of the other.

EPA 180327 (Amoco) describes an immunosassay system involving enzymes in which a reaction product can exist in a different (fluid) phase from the substrate.

The assay method of the present invention uses at least one, and in its preferred forms two or even more, enzyme amplification steps. In its preferred forms also, the method requires use of reagents which are readily obtained or prepared and which are stable for useful periods.

The invention provides a method of assaying for an analyte in a sample, by the use of a first enzyme, and a first substrate on which the first enzyme can act to generate a first product, which method comprises the steps:-

a) subjecting the sample to an assay procedure to generate the first enzyme in a predetermined state in an amount related to the concentration of the analyte in the sample,

b) contacting the first enzyme in the predetermined state with the first substrate under conditions to generate the first product in an amount related to the concentration of the analyte in the sample,

c) determining the amount, or the presence, of the first product by contacting the first product generated in step b) with a specific binder for the first product under conditions to cause them to become bound together, and

d) using the result of c) to determine the concentration, or the presence, of the analyte in the sample.

The nature of the analyte is not critical. The analyte may for example be a hapten or an antigen or an antibody, such as a hormone, a steroid, a drug or drug metabolite, a protein, a nucleic acid, a vitamin or a polysaccharide.

The nature of the sample is also not critical. The sample may be a biological fluid, such as serum, plasma, urine or milk.

The function of the first enzyme is to act on the first substrate so as to produce a distinctive first product. Examples can, in principle, be found in all the major classes of enzymes. Thus, the first enzyme may be an oxidoreductase, a transferase, a lyase, an isomerase, a ligase or a hydrolase. Within this last class of enzymes can be found those of particular potential importance, e.g. the glycosidases, phosphatases, sulphatases, esterases and lipases.

The function of the first substrate is to be acted on by the first enzyme so as to generate a distinctive first product. Therefore, the choice of the first substrate is dictated by the choice of the first enzyme.

A key feature of the invention is this action of the first enzyme on the first substrate to reveal a first product having antigenic determinants (or other binding domains) for binding to an antibody (or other specific binder) which may be labelled with a (component of a) signal generating system.

A principal function of the labelled specific binder is to bind specifically to the first product. The first product and the specific binder may form an immune pair, with one being the antigen and the other the antibody; or these two may constitute some other specific binding system, such as the avidin or streptavidin-biotin system. It is important that the labelled specific binder reacts, not significantly and preferably not at all, with the first substrate. Thus, the first product needs to be distinctive in the sense that it is recognized for binding purposes by the specific binder as distinct from the first substrate.

The label of the labelled specific binder may be any label conventionally used for detection in competition assays. For example, the label may be a radioisotope, a fluorescent material or a member of an enzyme system to generate a luminescent or electrochemical or colorimetric signal. Preferably, however, the label is a second enzyme, which may be the same as, or more preferably different from, the first enzyme, for by this means a second amplification stage can be introduced into the assay as will be described in more detail below.

The first step of the method involves subjecting the sample to an assay procedure to generate the first enzyme in a predetermined state in a concentration related to the concentration of the analyte in the sample. For this purpose, the enzyme is attached to the analyte itself or to one of the assay reagents, generally either an analogue of the analyte or a specific binder (e.g. an antibody) to the analyte. The attachment of enzymes to reagents of this kind, without loss of enzymatic properties, is well described in the literature. The predetermined state in which the enzyme is generated may conveniently be in solution (in which state it may readily be separated from enzyme bound to a solid surface); or alternatively bound to a solid surface (in which state it may readily be separated from enzyme in solution).

Several assay procedures for step a) of the method of the invention will now be described by way of example. A skilled reader will have no difficulty in concocting others. In these examples, it will be assumed for simplicity that the analyte is antigenic and that its specific binder is an antibody, although, as explained above, the invention is not so limited.

A. The reagents are an analyte analogue, in a matrix-bound form; and an antibody to the analyte, labelled with an enzyme, in solution. In a first step, the enzyme-labelled antibody in solution is contacted with the matrix-bound analyte analogue, to which it becomes bound. Excess solution is removed by washing. Then the liquid sample is incubated with the matrix-bound analyte analogue/antibody complex. Analyte in the sample competes with matrix-bound analyte analogue for binding to the antibody. As a result, there is released into solution an analyte/enzyme-labelled antibody complex, in a concentration directly proportional to the concentration of the analyte in the sample. This solution is then used for the next stage of the method.

There remains a matrix-bound analyte analogue/labelled antibody complex, in a concentration inversely proportional to the concentration of the analyte in the sample. It is alternatively possible to discard the solution resulting from the incubation and to the use for step b) this matrix bound-material instead.

B. The reagents are a matrix-bound antibody to the analyte; and an enzyme-labelled analyte analogue in solution. In a first step, the enzyme-labelled analyte analogue in solution is contacted with the matrix-bound antibody, and becomes bound thereto. Excess solution is removed by washing. Then the sample is incubated with the matrix-bound antibody/labelled analyte analogue complex. Analyte in the sample competes with labelled analyte analogue for binding to the antibody, and displaces some of the labelled analyte analogue into solution. The resulting solution, which contains enzyme-labelled analyte analogue in a concentration directly proportional to the analyte concentration in the sample, can be used for step b).

Alternatively, as in A, the solution can be discarded and the solid phase used instead.

C. The reagents are the same as in A, but the order of reaction is different. In a first step, the sample is incubated with the solution of enzyme-labelled antibody. In a second step, the resulting mixture is contacted with the matrix-bound analyte analogue.

Alternatively, the sample and the enzyme-labelled antibody solution could have been added to the matrix-bound analyte analogue at the same time and the whole procedure performed in a single incubation.

D. The reagents are the same as in B, but the order of reaction is different. In a first step, the sample is incubated with the enzyme-labelled analyte analogue solution. In a subsequent step, the resulting mixture is contacted with the matrix-bound antibody.

Alternatively, the sample and the enzyme-labelled analyte analogue solution could have been added to the matrix-bound antibody at the same time, and the whole procedure performed in a single incubation.

E. This alternative is possible only when the analyte is polyepitopic, having several sites available for immune binding. The reagents are a matrix-bound first antibody to the analyte; and an enzyme-labelled second antibody to the analyte in solution. The method involves incubating these two reagents with the sample, in one or two stages and with any order of mixing. The result is a sandwich of matrix-(first antibody)-(analyte)-(enzyme-labelled second antibody). The amount of enzyme-labelled second antibody on the solid phase is directly proportional to the amount of analyte in the sample. The amount of enzyme-labelled second antibody in solution is inversely proportional to the amount of analyte in the sample. Either the solution or the solid phase can be used for step b).

A function of the procedure of step a) is to separate the enzyme-labelled reagent into two fractions. However, it is not necessary that one of these be in the liquid phase while the other is matrix-bound. Indeed, physical separation of the two fractions is not always necessary. It is possible to devise a system in which the enzyme in one fraction, but not the other, has been inactivated. Other systems include the following.

F. Detection of micro-organisms where the micro-organism is trapped in a matrix, and enzyme-labelled antibody is applied.

G. Dipstick-like procedures in which an enzyme-labelled antibody or antigen is made to move to a different zone in response to the presence of an analyte.

Step b) of the method of the invention involves contacting the first enzyme in the predetermined state, i.e. the chosen fraction of the enzyme-labelled reagent, with an amount of a first substrate under conditions to generate a first product. When the first enzyme is in solution, and in order to facilitate the subsequent steps of the method, it will usually be convenient to provide the substrate in a form bound to a solid matrix or to some other material by means of which it may be readily separated from the supernatant liquid. The first substrate may comprise the first product masked by a group which is removed by action of the enzyme to reveal the first product.

Immobilized and immobilizable substrates, for this purpose can be prepared according to well established chemical methods. The general structure of such substrates can be represented as follows:-
X-O-A-L-M
where X is a glycosyl, phosphate, diphosphate, sulphate or other residue chosen in relation to the enzyme being used;
O is usually, but not necessarily, a hydroxy function, but may also be for example an amino, carboxyl or sulphydryl group;
A may be for example an aromatic nucleus, part of a heterocyclic centre, peptide or steroid, revealed by removal of X by the enzyme;

L is a chemical bridge formed by a bifunctional coupling agent such as glutaraldehyde, dihydrazide, amino acid, 6-aminohexanoic acid, diimidate; and
M may be a macromolecular carrier such as a carrier protein, soluble polysaccharide or enzyme; or a solid support such as a glass, plastic, polymer, ceramic or paper surface; or a particulate support such as polysaccharide or polymer; or a chemical substance such as a hapten or antigenic determinant.

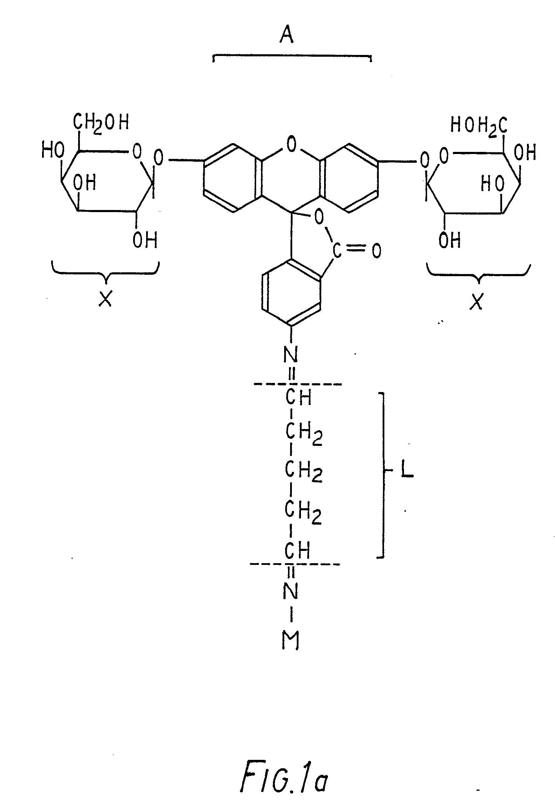

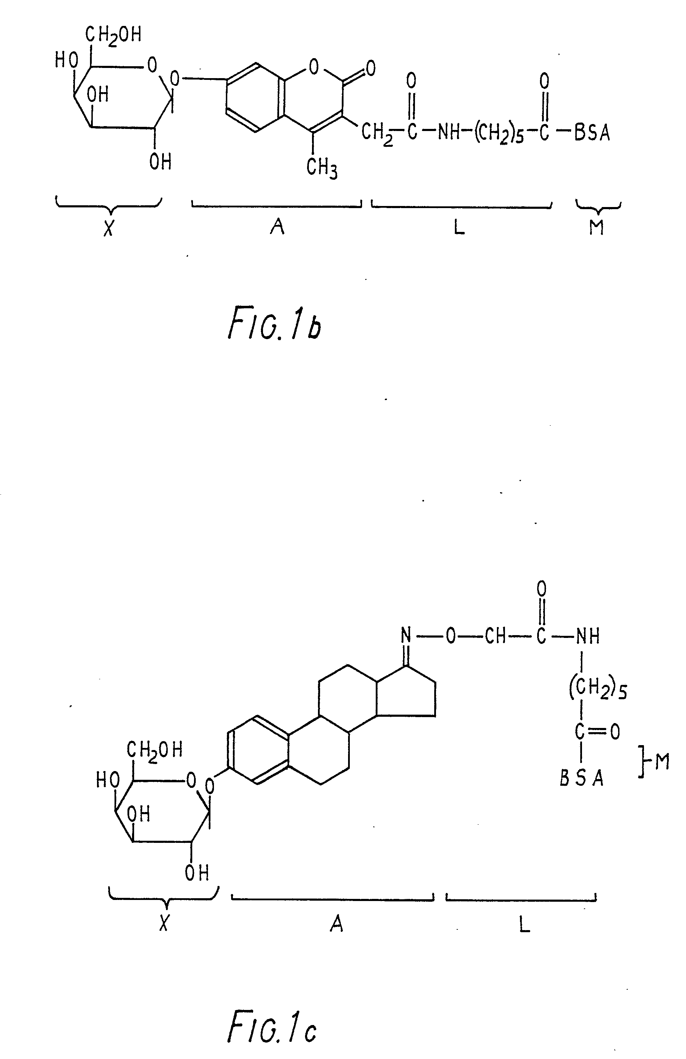

Reference is directed to the accompanying drawings, in which Figures 1a, 1b and 1c are examples of substrates according to the above nomenclature.

In Figure 1a:-
X is galactose
A is fluorescein
L is from glutaraldehyde
M is carrier protein e.g. Bovine serum albumin

In Figure 1b:-
X is galactose
A is 7-hydroxy-4-methyl-coumarin-3-acetic acid
L is 6-amino caproic acid
M is carrier protein e.g. Bovine serum albumin

In Figure 1c:-
X is galactose
A is oestrone
L is N-(O-carboxymethyl) oxime
M is carrier protein e.g. Bovine serum albumin

The introduction of glycosidic residues into the hydroxyl functions of A structures may be performed according to the general principles of the Koenigs-Knorr reaction. Tetra-O-acetyl-alpha-D-galactopyranosyl bromide may be reacted with the hydroxyl-A under appropriate conditions. Isolation of the final product can be effected by standard chemical procedures using acid/alkaline extraction steps as well as column chromatography on silica gel. The preparation of first substrate, both in solution and in matrix-bound form, is described below in Examples 1 - 3, and EPA 28332 also provides relevant information.

An advantage of providing the first substrate in a matrix-bound form is that steps a) and b) of the assay method can be performed in a single vessel without the need for decanting or washing. A disadvantage is that the kinetics of reaction between the matrix-bound substrate and the first enzyme may be rather slow. In cases where this proves a problem, it may be preferred to provide the first substrate in solution. In that case, the first enzyme may be generated either in solution or in a matrix-bound form. It is often more convenient to generate the first enzyme in a matrix-bound form.

The first enzyme in the predetermined state is incubated with the first substrate to reveal the first product. This is the first amplification stage of the method. The amount of the first substrate and the time and other conditions of incubation need to be chosen together to ensure that the first product is revealed at a concentration which is not only amplified but also related to the concentration of the analyte in the starting sample. In other words, reaction between the first enzyme and the first substrate must not be allowed to go to completion, so that there is left at the end of the step a mixture of the first substrate and the first product.

In step c) of the method, the first product is contacted with a labelled specific binder under conditions to cause them to become bound together, and in this way the amount, or the presence, of the first product is determined. How this is done is not material to the invention. Many different techniques are available and described in the literature. When the first substrate has been provided in a matrix-bound form, this step usually involves incubating the labelled specific binder with a mixture of the first product and unreacted residual first substrate. As noted above, it is important to the success of the method that the labelled specific binder should bind, not significantly and preferably not at all, to the first substrate. Design of the first product and first substrate for this purpose is to some extent a matter of trial and error, albeit in accordance with known principles. Using a polyclonal antibody as the specific binder, we have achieved cross reactivities below 0.018%, and expect that this figure would be reduced much further by the use of a monoclonal antibody.

When the first substrate is provided in solution, with the result that the first product is generated in solution, it is preferable to separate the two before going on to step c). This may be done by the use of excess of a matrix-bound antibody (or other specific binder) to bind to the antigenic determinants (or other binding sites) of the first product that have been revealed by action of the first enzyme on the first substrate. The supernatant liquid comprising residual first substrate is decanted and the solid phase washed. Then, preferably by the use of another antibody (or other specific binder) labelled with a (component of a) signal generating system, a labelled reagent is caused to become bound to the matrix-bound first product.

After the step c) incubation, it is normally necessary to separate that portion of the labelled specific binder that has become bound to the first product, from that portion not so bound. If the first product is immobilised on a solid matrix, such separation is readily achieved by decanting and washing. If the first product is generated in solution, then some other means of separation may need to be found. Alternatively, for some label systems physical separation may not be necessary, for example, label bound (or not bound) to the first product may be masked or inactivated in some way.

It is necessary to determine the label bound, or not bound, to the first product. For a quantitative assay, this involves determining the concentration of the bound or unbound label. Techniques for doing this are well known in the art, depending on the nature of the label used, and will not be described here. In a preferred mode of operating this invention, when the label is a second enzyme (different from the first enzyme and not reactive with the first substrate), this determination involves a second amplification stage. Thus, one molecule of bound second enzyme acts on many molecules of a second substrate in the solution to generate many molecules of a second product which is observed, e.g. by luminescence, fluorescence or colour change. In these circumstances, the second substrate needs to be provided in excess, and incubation with the bound enzyme needs to be continued under predetermined conditions for a predetermined time.

Step d) of the method involves using the result of step c) to determine the concentration, or the presence, of the analyte in the sample. This is generally done by means of a standard curve. Standard samples containing known concentrations of analayte spanning the expected range of the unknowns are subjected to the method. The results are used to plot a graph of signal intensity against analyte concentration. After the signal intensity of an unknown sample has been determined, the analyte concentration in that sample can simply be read off the graph.

The following examples illustrate the invention.

### Example 1

#### Preparation and Properties of Di-galactosyl Fluorescein Amine-Hemisuccinate

Fluorescein amine (1g, 2.9 mmoles) and succinic anhydride (400 mg, 4 mmoles) were refluxed in 50ml THF, for 4 hours. After removal of the solvent, 100ml of 0.5M HCl was added to the residue. After 1 hour on ice, 200ml ethyl acetate was added in order to dissolve the heavy precipitate which had appeared.

The organic layer was washed with acid, with water, dried over $Na_2SO_4$, and reduced in volume until the fluorescein amine-hemisuccinate (FAHS) began to precipitate. Precipitation was allowed to continue in the cold, after which the product was washed with cold ethyl acetate and dried under vacuum.

Fluorescein amine hemisuccinate (500mg, 1.1 mmole) and acetobromo-alpha-D-galactose (2g, 4.9 mmole) were refluxed for 24 hours in a mixture of dry acetone (50ml) and methanol (5ml) in the presence of 4g of anhydrous $K_2CO_3$. The reaction was monitored by TLC in ethyl acetate: MeOH (80:20).

Following reaction, the mixture was cooled, filtered and the solvents were removed. The residue was then washed with dry ethyl acetate: diethyl ether mixture (50:50) to remove the excess bromosugar. The residue was taken up into cold 0.1M $NaHCO_3$, washed with ethyl acetate, acidified while on ice, extracted into ethyl acetate, and washed with water. The organic layer was then dried, and after the solvents had been removed, the mixture was left in a desiccator under vacuum overnight. To the resulting oily residue dissolved in 15ml methanol A.R. cooled on ice, was added 200μl 1M sodium methoxide, and after 1 hour 100ml of ethyl acetate: diethyl ether (50:50) mixture. This was left in the cold overnight in order for the product to precipitate. The precipitate was washed several times with cold ether and ethyl acetate, and then dried. This material was almost pure substrate. Traces of fluorescein were removed by the extraction of an acidified aqueous solution of the substrate with ethyl acetate. The cleaned product was neutralised and then freeze dried.

Prior to its assessment as a beta-galactosidase substrate, the di-galactosyl fluorescein amine-hemisuccinate was examined by scanning spectrophotometry in order to identify the wavelength of its maximum absorbance. Using stock solutions of known concentration, a preliminary molar extinction coefficient for the aglycone at 490nm was determined of approx. 38,000. Inspection under U.V. illumination showed a very pronounced difference between the fluorescence of the aglycone and the much less fluorescent di-galactoside.

In order to demonstrate hydrolysis of the substrate, a time course experiment was performed using beta-galactosidase at pH 7.3, and a final substrate concentration of 2.7mM. The increase in absorbance at 490nm was monitored in order to measure aglycone formation.

Substrate (200μl; 4mM in 50mM Tris buffer pH 7.3, 0.1M NaCl, lmM $MgCl_2$) and E. coli beta-galactosidase (100μl; 0.28U) were incubated at 37°C for various periods of time. The reaction was stopped by the addition of ice cold 0.5M $CO_3/HCO_3$ buffer pH 9.5 (0.5ml) and the absorbance of the reaction mixture read at 490nm. The slope of the line generated indicated that 74 pmoles of product was produced per minute under these conditions.

Since the di-galactosyl-FAHS showed clear activity as a beta-galactosidase substrate, the cross-reactivity of this substrate with respect to its aglycone was assessed using the $^3$H-fluorescein tracer. The results showed that while the anti-fluorescein antiserum was easily able to recognise and bind the aglycone, it was less easily able to recognise and bind the intact substrate.

A 45 fold molar excess of FAHS was required to inhibit the binding of the $^3$H-tracer by a suitable diluted anti-fluorescein antiserum by 50%, while over the range tested, the di-galactosyl-FAHS was unable to inhibit this binding. The cross reactivity of the substrate was therefore less than 0.45% of that exhibited by the aglycone.

Example 2

Coupling of Di-galactosyl-fluoresceinamine-HS to AH-sepharose

One gram of AH-sepharose 4B was swollen, washed and suspended in 10ml of 0.01M phosphate buffer, pH 6.8. To the suspension was added 209mg substrate from Example 1 and 400mg CMC, and the mixture was mixed with rolling for 24 hours. A further 200mg of CMC was added, and the reaction was allowed to proceed for a further 24 hours. The gel was washed using the usual conditions of low and high pH, and then stored in PBS. It had a pronounced yellow colour. To test whether the substrate on the sepharose was available for enzymic hydrolysis, a series of 20μl aliquots of a 50% suspension of substrate-sepharose were incubated with or without beta-galactosidase, overnight. RIA performed in the same tubes showed that the test samples gave a 27% binding of tracer while the control samples bound 45% of the tracer. This result indicated clearly that the substrate on AH-sepharose was available for hydrolysis by the enzyme.

Example 3

Mono-galactoside-fluorescein substrates

Nitro-fluorescein was prepared according to standard methods. The methyl ester was prepared by refluxing nitrofluorescein in excess methanol in the presence of 1% conc. $H_2SO_4$.

The galactose was introduced into the remaining free hydroxy function of nitrofluorescein-methyl ester using tetra-acetyl-bromogalactose in the presence of silver carbonate (catalyst) and calcium sulphate (internal desiccant) in benzene-tetrahydrofuran mixture.

The methoxy derivative of nitrofluorescein methyl ester was prepared using dimethyl sulphate and 0.lN NaOH according to standard methylation procedure. Removal of the ester gave mono-methoxy nitrofluorescein. The galactose was introduced as given above.

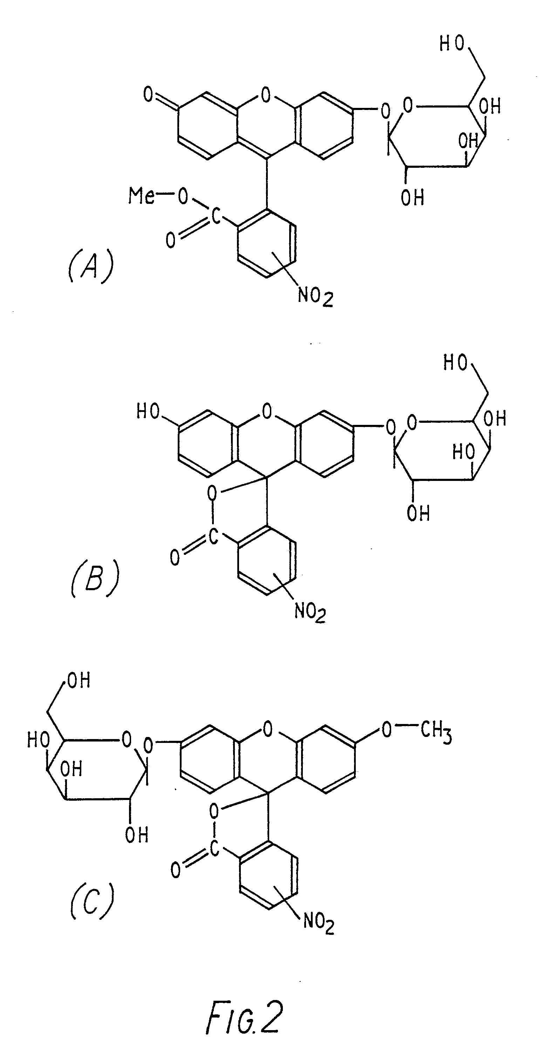

Three possible mono-galactoside nitrofluorescein derivatives can result from the steps outlined; (A), (B) and (C). (See Figure 2).

Reduction of the nitro-function allows further modification through the resulting amino group.

Example 4

Preparation of an Ouabain-Fetuin Conjugate

An ouabain-fetuin conjugate was prepared using the periodate oxidation/ Schiffs base procedure. Ouabain (200 mg) containing 10.8 $\mu$Ci $^3$H-digoxin was oxidised with sodium metaperiodate for 2h at room temperature, and then reacted with 110 mg Fetuin at pH 9-9.5 for a further 2h at room temperature. The reaction product was stabilised by reaction with 137 mg sodium borohydride for 4h, dialysed against water for 6 days (5 changes, 5 litres), lyophilised to reduce the volume, and finally subjected to gel filtration on Sephadex G50 to remove any remaining traces · of unconjugated ouabain.

Example 5

Demonstration of the Complete Protocol for an Amplified Immunoassay

An ELISA format was chosen to demonstrate the complete sequence of reactions based on the amplification protocol. The synthesis of the ouabain-fetuin conjugate employed below was described in Example 4.

In preparation for the assay, two PVC ELISA plates were pre-coated. In the first (Plate 1) the wells of the plate were coated overnight at 4°C with either ouabain-fetuin or fetuin alone (10 $\mu$g in 100 $\mu$l coating buffer). The plate was then washed three times with washing buffer, and to each well was added $\beta$-galactosidase-conjugated anti-digoxin IgG (100 $\mu$l; conjugate from above diluted 1 + 1 with washing buffer). After 2 hours at 37°C excess antibody-enzyme conjugate was washed from the plate (3 washes with washing buffer).

The wells of the second plate (Plate 2) were coated overnight at 4°C with either BSA alone (100 $\mu$l; 1 mg/ml in coating buffer) or BSA-coumarin-$\beta$-galactoside (100 $\mu$l;1 mg/ml in coating buffer). Immediately before use, the plate was washed three times in coating buffer, and a further once with $\beta$-galactosidase assay buffer (0.1M sodium phosphate buffer pH 7.1, 0.1M NaCl, 10 mM MgCl$_2$).

The assay was performed as follows. To each well of Plate-1 containing ouabain-fetuin antigen was added digoxin solution (1 ng/ml - 1 mg/ml in 150 $\mu$l PBS). After 1 hour at 37°C, the contents of each individual well were mixed, and a sample of the supernatant from each (100 $\mu$l) transferred to a well of Plate-2 containing immobilised BSA-coumarin-$\beta$-galactoside (and 50 $\mu$l $\beta$-galactosidase assay buffer). PBS (100 $\mu$l) was placed in wells of Plate-2 containing immobilised BSA (and 50 $\mu$l $\beta$-galactosidase assay buffer).

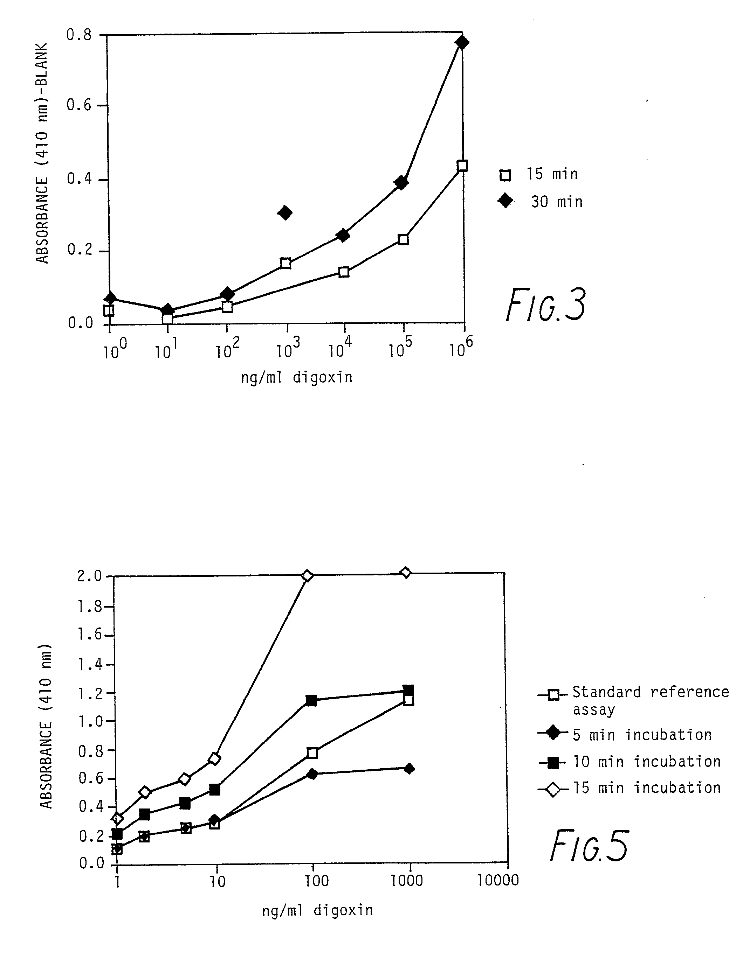

After 2hours at 37°C, Plate-2 was twice washed with washing buffer, and alkaline phosphatase-conjugated anti-coumarin IgG (100 $\mu$l; 1 in 4 diluted with washing buffer) added to each well. The plate was incubated for 1.5 hours at 37°C, after which time the unbound antibody-enzyme conjugate was removed from the plate wells by washing (3 times) with washing buffer. Alkaline phosphatase substrate solution (100 $\mu$l; 6 mM pNp-phosphate in 0.1M glycine buffer pH 10.4, 1 mM ZnCl$_2$, 1 mM MgCl$_2$) was added to each well, and the plate incubated at room temperature. After 15 minutes and then 30 minutes, the plate was read at 410 nm in an ELISA plate reader (Figure 3).

Figure 3 shows the activity of bound alkaline phosphatase-conjugated anti-coumarin IgG to immobilised coumarin residues following their exposure by hydrolysis of immobilised coumarin-$\beta$-galactoside by displaced $\beta$-galactosidase-conjugated anti-digoxin IgG. The binding of alkaline phosphatase-anti-coumarin to BSA alone was less than 5% of maximal binding to BSA-coumarin. The binding of the conjugate to unhydrolysed substrate was approximately 29% of maximal bnding. This value was subtracted from all others before plotting. Assays were performed in duplicate.

These results now constitute the basis of an assay for digoxin in which the signal is directly proportional to the concentration of the analyte applied, over the range 10 ng/ml to 1 mg/ml.

Example 6

Experimental Method

### 1. Preparation of a Solid-Phase Catch System.

To each well of the PVC ELISA plate was added approximately 10 μg of affinity purified anti-coumarin IgG in 100 μl coating buffer, and the plate was incubated in a moist atmosphere overnight at 4°C. The following day, unbound IgG was washed from the plate with washing buffer (4 wash cycles).

### 2. Coumarin substrate-estradiol double conjugate on dextran-6-amino hexane

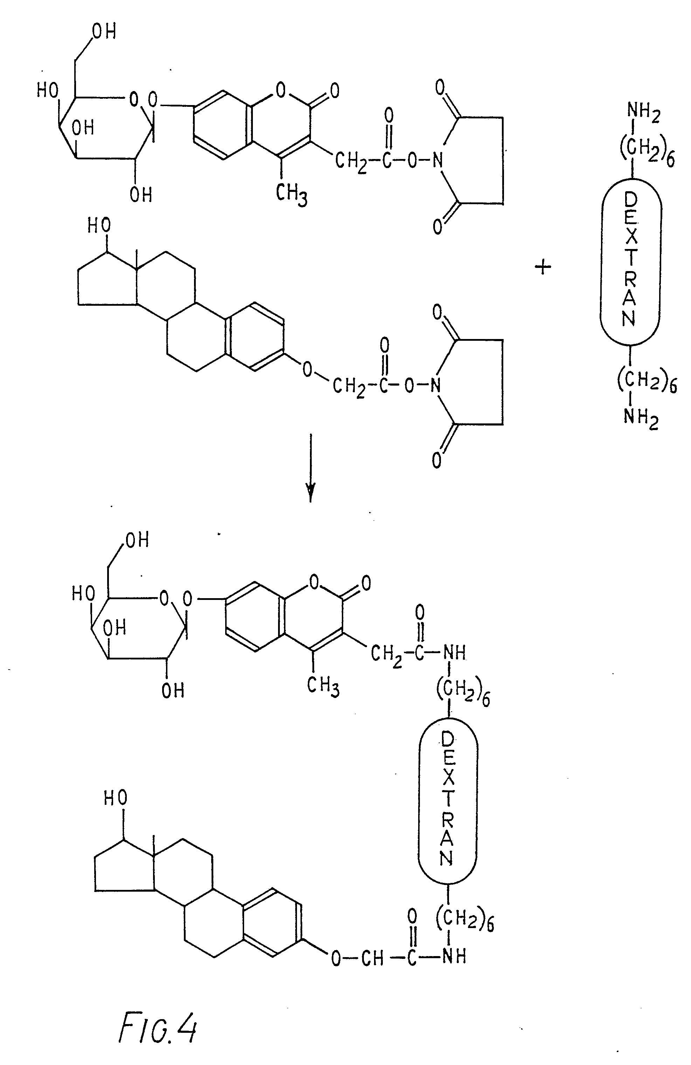

Dextran T40 was oxidised by sodium periodate to produce aldehyde functions; diamino hexane was introduced followed by reduction with sodium borohydride to stabilize the link. Coumarin-galactose and estradiol were introduced into the dextran-6-amino hexane via active esters of their derivatives. The active esters were added sequentially to the dextran to a ratio of 5:1 substrate to estradiol. The reaction is shown in Figure 4.

The double conjugate was dialysed and chromatographed on Sephadex G50. The fractions containing the dextran showed fluorescence (faint, indicative of the coumarin substrate).

### 3. Preparation of Dextran-Substrate Solution

A solution of the resulting dextran-substrate (1 mg/ml) in β-galactosidase assay buffer was prepared, and to this was added anti-coumarin IgG (12 μl suitably diluted anti-coumarin IgG in washing buffer/ 120 μl dextran-substrate) to block any exposed sites which may be present as impurities. (This step is not obligatory to the method). The mixture was incubated for 1 hour at 37°C.

### 4. Preparation of Solid-Phase Ouabain-Anti-Digoxin-β-Galactosidase Conjugate

To each well of a PVC ELISA plate was added approximately 10 μg of fetuin-ouabain (Example 4) in 100 μl coating buffer, and the plate was incubated in a moist atmosphere overnight at 4°C. Unbound conjugate was washed from the plate with washing buffer (4 wash cycles). To each well was then added β-galactosidase-conjugated affinity-purified sheep anti-digoxin IgG (4 μg conjugate in 100 μl PBS 3 mg/ml BSA) (anti-digoxin-conjugate).

The plate was then incubated at room temperature for 2 hours, and excess conjugate removed from the wells by washing, as above.

### 5. Standard and Amplified Assays Employing Galactosidase Activity Generated by Displacement with Digoxin in Solution.

Digoxin solutions (1 ng-1 μg/ml in PBS) or PBS alone, were prepared, and 100 μl of each added to the wells of an ELISA plate displaying solid-phase ouabain-anti-digoxin conjugate. After 60 minutes at room temperature, with agitation, 100 μl from each well was either assayed using oNp-galactoside in a standard reference assay, or added to 100 μl dextran-substrate.

After 20 minutes, 100 μl of each reaction mixture was transferred to the wells of the ELISA plate with anti-coumarin coated wells, and this plate was then incubated for a further 20 minutes. Unbound reaction product was then washed from the wells with washing buffer (4 wash cycles) and alkaline phosphatase-conjugated affinity-purified anti-oestradiol IgG (100 μl at 5 μg/ml) was added to each well.

After a further 20 minutes, unbound conjugate was washed from the wells as above, and alkaline phosphatase substrate solution (100 μl; 6 mM pNp-phosphatase in phosphatase assay buffer) was added to each well.

The absorbance of the product solutions were read after 5, 10 and 15 minutes, against the substrate blank (100 μl alkaline phosphatase substrate solution incubated over the same period).

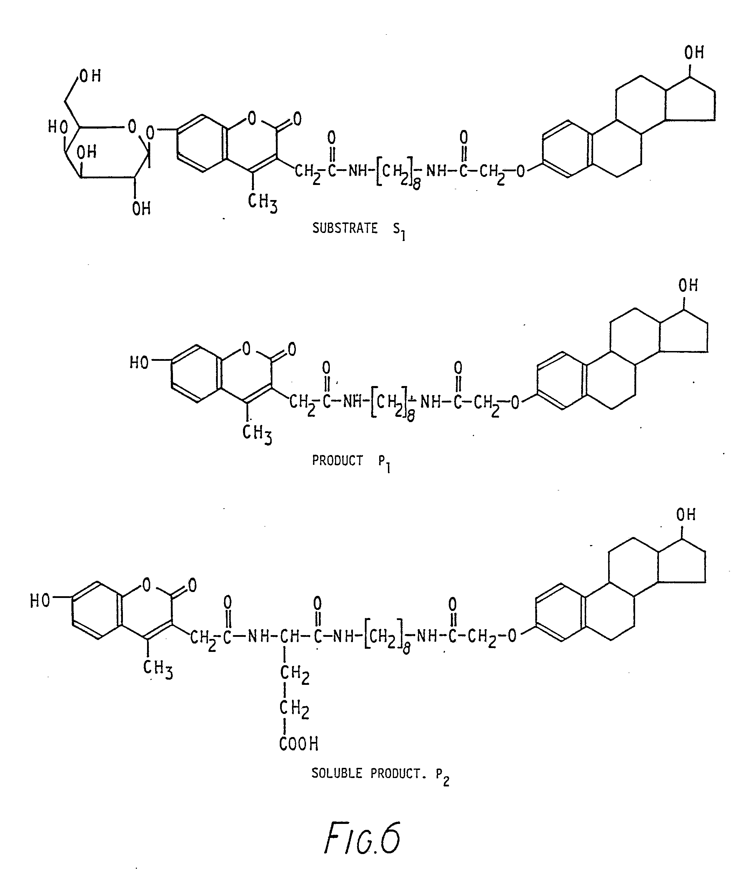

The results are shown in Figure 5. It should be noted that in the standard reference oNp assay, incubation was continued for 90 minutes. Provided the incubation time for signal generation was at least 10 minutes, the assay of the invention was more sensitive than the standard reference assay.

### Example 7

### Estimation of the cross reaction of the coumarin-galactosyl substrate

Galactosyl-coumarin-3-acetic acid was prepared according to standard chemical procedure. The cross reaction was estimated by radio-immunoassay using 3H-labelled 7-hydroxy coumarin-3-acetic acid derivative and rabbit anti-7-hydroxy coumarin-3-acetic acid.

The standard dextran coated charcoal method was employed, and the commonly used 50% inhibition formula was used to calculate the cross reaction figure, = 0.008 - 0.018 %. The low cross reaction indicates the large difference in affinities of the coumarin and the galactosyl-coumarin for the anti-coumarin antibodies.

## Example 8

### Preparation and Properties of Coumarin-Galactosyl Substrate Estradiol (haptenic moiety) Conjugates

Conjugates of coumarin-3-acetic acid and 17β-estradiol-3-carboxymethyl ether with 1,8-diamino octane as an arm was prepared and purified by standard organic chemistry procedures. A soluble version of the conjugate was also prepared (see Figure 6). Galactose was introduced on to the 7-hydroxy function of the coumarin in the conjugate by standard methods.

The immune activities of the conjugate and the substrate conjugate were analysed by standard ELISA procedure.

Microtitre plates were coated with rabbit anti-coumarin. A range of concentrations of the conjugate and the substrate were added to the coated wells, allowed to react, and after washing anti-estradiol antibody labelled with alkaline phosphatase was added to the wells and was allowed to react.

After washing, the alkaline phosphatase activities retained by the wells were measured by using pNp phosphate.

The results showed the marked difference of the reactivities of the substrate and the product. The much lower affinity of the substrate S1 for the anti-coumarin antibody is due to the masking of the antigenic determinant (the coumarin moiety). Exposure of the antigenic determinant by the removal of the galactose $(S_1 \rightarrow P1)$ increased the affinity by a factor of about 1000. The reactivity of the more soluble version of the conjugate (S2 - P2) was slightly better. The figures were confirmed by the cross reaction data.

## Example 9

### Assay of β-galactosidase using standard procedure and the amplification method

A range of dilutions of E.Coli-β-galactosidase were prepared from a stock solution (about 1mg/ml of a commercial preparation).

The standard assay was carried out as follows:-
In the microtitre plate wells, 10 μl of β-galactosidase were added to 100 μl of pNp-galactoside and after 90 minutes the reaction was terminated by 100 μl stopping buffer and the absorbance levels were read.

The amplified assay was carried out as follows:-
In the microtitre plate wells, 10 μl of the enzyme were added to 100 μl of 50 μg/ml of galactosyl-coumarin diamino octane-estradiol 3 CME conjugate. After 20 minutes incubation, the reaction products were diluted 1/256 and 100 μl were transferred to microtitre plate wells coated with anti-coumarin. After 20 minutes the plate was washed and anti-estradiol-alkaline phosphatase was added and allowed to react for 20 minutes before washing and phosphatase activity measurements at from 10 to 30 minutes.

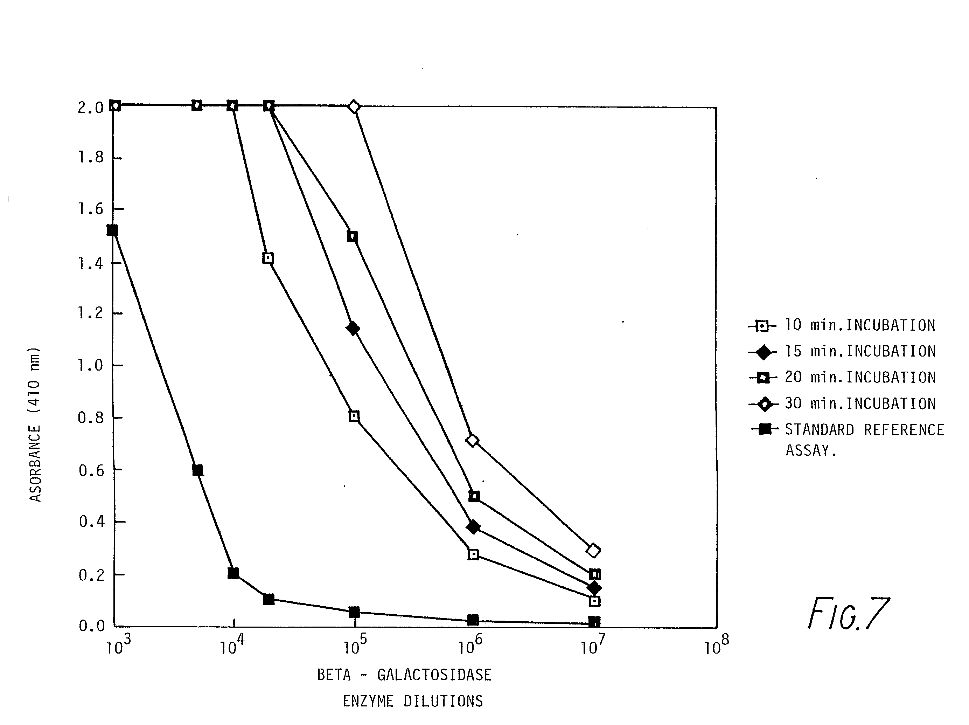

The graph (Figure 7) shows the absorbances against β-gal dilutions. The amplified assay is about 1000 times more sensitive than the standard assay (see above). (Not taking into account the dilution factor of the product prior to the introduction to the anti-coumarin antibody coated wells).

## Example 10

### Enzyme-immunoassy of 17α-hydroxyprogesterone (competitive assay procedure)

Comparison between a standard assay procedure and the amplified method.

### Reagents and steps:

Sheep anti 17-OH-Progesterone was coupled to particles by the standard CNBr activation method.

17-OH-Progesterone-3-carboxymethyl oxime was prepared and conjugated to E.Coli β-galactosidase (commercial preparation) by standard methods.

A competitive enzyme immunoassay was established (free standard analyte competes with the enzyme-labelled analyte for the antibody on the magnetizable particles). The activity of the bound fraction of tracer was measured using the standard pNp-galactoside or the amplification procedure using galactosyl-coumarin-diamino octane-estradiol 3 CME as a first substrate and magnetizable particles coupled anti-coumarin preparation to capture product 1 (or a fraction of it). The levels of the captured product 1 were estimated by the use of anti-estradiol alkaline phosphatase and conjugate and a phosphatase activity measurement using pNp-phosphate.

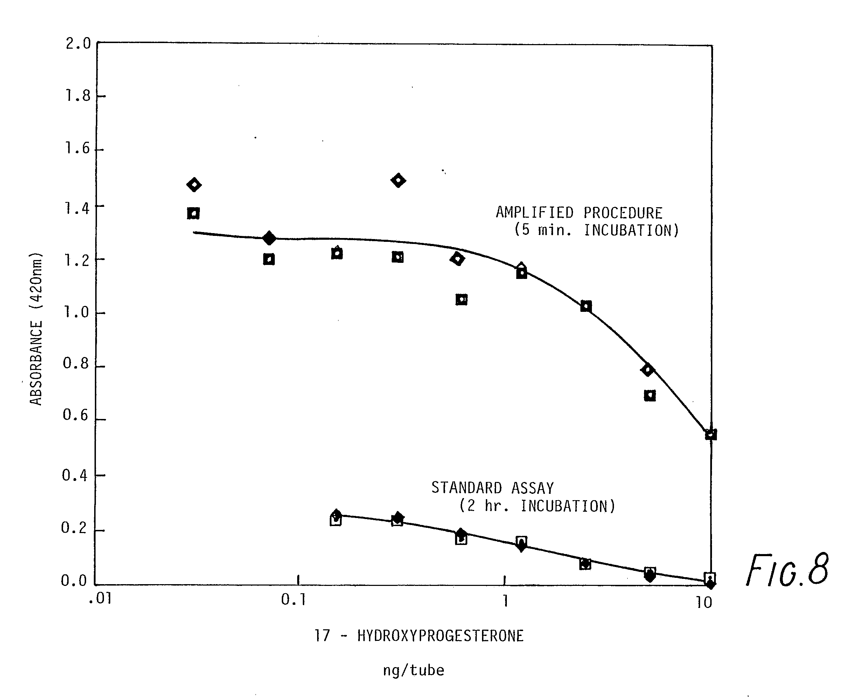

An example of the results obtained is presented on Figure 8. The amplified procedure gives a very large increase in the signal even though only a small fraction of P1 was carried through to the second stage of the assay.

## Claims

1. A method of assaying for an analyte in a sample, by the use of a first enzyme, and a first substrate on which the first enzyme can act to generate a first product, which method comprises the steps:-

a) subjecting the sample to an assay procedure to generate the first enzyme in a predetermined state in an amount related to the concentration of the analyte in the sample,

b) contacting the first enzyme in the predetermined state with the first substrate under conditions to generate the first product in an amount related to the concentration of the analyte in the sample,

c) determining the amount, or the presence, of the first product by contacting the first product generated in step b) with a specific binder for the first product under conditions to cause them to become bound together, and

d) using the result of c) to determine the concentration, or the presence, of the analyte in the sample.

2. A method as claimed in claim 1, wherein the first substrate is provided in a matrix-bound form, whereby the first product is generated in step b) in a matrix-bound form.

3. A method as claimed in claim 1, wherein the first substrate is provided in solution, whereby the first product is generated in step b) in solution.

4. A method as claimed in claim 3, wherein the first enzyme is generated in step a) in a matrix-bound form.

5. A method as claimed in claim 3 or claim 4, wherein the first product generated in step b) is converted from solution to a matrix-bound form prior to performance of step c).

6. A method as claimed in any one of claims 1 to 5, wherein step c) is performed by contacting the first product with a labelled specific binder for the first product under conditions to cause them to become bound together, and determining the label bound, or not bound, to the first product.

7. A method as claimed in claim 6, wherein the specific binder is labelled with an enzyme.

8. A method as claimed in any one of claims 1 to 7, wherein the first enzyme is beta-galactosidase.

9. A method as claimed in claim 8, wherein the first substrate is di-galactosyl-fluorescein amine-hemisuccinate or a mono-galactoside-fluorescein substrate.

10. A method as claimed in claim 8, wherein the first substrate is a a galactosyl-coumarin-estradiol substrate.

11. A method as claimed in any one of claims 1 to 10, wherein there is provided an analyte analogue in matrix-bound form and an enzyme labelled analyte-antibody in solution, and the assay procedure of step a) involves causing analyte in the sample to compete with the matrix-bound analyte analogue for binding to the enzyme-labelled antibody.

FIG.1a

FIG.1b

FIG.1c

FIG.2

FIG.3

FIG.5

FIG.4

SUBSTRATE $S_1$

PRODUCT $P_1$

SOLUBLE PRODUCT. $P_2$

*FIG.6*

_FIG.7_

- 10 min.INCUBATION
- 15 min.INCUBATION
- 20 min.INCUBATION
- 30 min.INCUBATION
- STANDARD REFERENCE ASSAY.

BETA - GALACTOSIDASE ENZYME DILUTIONS

ASORBANCE (410 nm)

FIG.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| A | EP-A-0 180 327  (AMOCO CORP.) * Page 5, line 5 - page 6, line 13; page 35, line 1 - page 36, line 2; claims * | 1 | G 01 N  33/58 G 01 N  33/543 G 01 N  33/74 G 01 N  33/533// G 01 N  33/94 |
| A | EP-A-0 152 254  (E.I. DU PONT DE NEMOURS & CO.) * Page 8, line 6 - page 13, line 13; page 19, line 10; claims * | 1 | |
| A | EP-A-0 075 379  (SYVA CO.) * Abstract; page 1, line 1 - page 5, line 31; page 15, line 8 * | 1 | |
| A | EP-A-0 028 332  (MILES LABS) | | |

TECHNICAL FIELDS
SEARCHED (Int. Cl.4)

G 01 N

The present search report has been drawn up for all claims

| Place of search | Date of completion of the search | Examiner |
|---|---|---|
| THE HAGUE | 30-11-1988 | HITCHEN C.E. |